# EUROPEAN PATENT SPECIFICATION

(11) **EP 3 741 209 B1**
(45) Date of publication and mention of the grant of the patent: **28.12.2022**
(21) Application number: 20174313.5
(22) Date of filing: 13.05.2020
(51) Int. Cl.: A01K 23/00, A61F 13/514, A61F 13/15

(54) **DISPOSABLE DIAPER FOR PET AND METHOD FOR MANUFACTURING DISPOSABLE DIAPER FOR PET**
WEGWERFWINDEL FÜR HAUSTIERE UND VERFAHREN ZUR HERSTELLUNG EINER WEGWERFWINDEL FÜR HAUSTIERE
COUCHE JETABLE POUR ANIMAL DE COMPAGNIE ET PROCÉDÉ DE FABRICATION D'UNE COUCHE JETABLE POUR ANIMAL DE COMPAGNIE

(30) Priority: 20.05.2019 JP 2019094869; 11.11.2019 JP 2019203994
(43) Date of publication of application: 25.11.2020
(73) Proprietor: Unicharm Corporation, Shikokuchuo-shi, Ehime 799-0111 (JP)
(72) Inventor: KOMATSUBARA, Daisuke, Kanonji-shi, Kagawa 769-1602 (JP)
(74) Representative: Staeger & Sperling Partnerschaftsgesellschaft mbB

(56) References cited:
- EP-A1- 3 476 213
- US-A1- 2010 319 633

## Description

### TECHNICAL FIELD

The present invention relates to a disposable diaper for pet placed on a pet such as dog and cat.

### BACKGROUND ART

Patent Literatures 1 discloses disposable diapers for pet. The disposable diaper for pet disclosed in Patent Literature 1 a band-like main body having an absorbent core, an adhesive seal arranged on the top face side of the main body, and a seal receiving part arranged on the back face side of the main body. When the disposable diaper for pet disclosed in Patent Literature 1 is placed, the disposable diaper for pet is positioned so as to align the longitudinal direction of the main body to the waistline direction of a pet, and to cover the urethral opening of the pet with the absorbent core, thereby wrapping around the waist of the pet with the aid of the adhesive seal and the seal receiving part (see Fig. 3 of Patent Literature 1).

### CITATION LIST

### PATENT LITERATURE

Patent Literature 1: JP 3141580U. Further prior art in this technical field defining the preamble of claim 1 is disclosed by documents EP 3 476 512 A1 and US 2010/319633 A1.

The disposable diaper for pet described in Patent Literature 1 has, however, suffered from a problem below.

The longitudinal direction of the main body of the disposable diaper for pet is laid around the waistline direction of the pet, and the transverse direction is disposed along the front-back direction that extends from the head side to the crotch side. Now, it is a general practice to put the disposable diaper for pet equally over the entire range of the front-back direction. The waist size of the pet, however, varies in the front-back direction. Hence, a gap has occasionally been produced between the waist of the pet and the disposable diaper, degrading the fitting quality.

It is therefore an object of the present invention to provide a disposable diaper for pet, which can be placed conforming to the waist shape of pet, and can improve the fitting quality.

A disposable diaper for pet according to one embodiment includes : a waistline direction disposed along a waistline direction of a pet; a cross direction orthogonal to the waistline direction; a main body portion having a topsheet, a backsheet, and an absorbent core arranged between the topsheet and the backsheet; and a binding portion that binds opposing parts of the main body portion when wrapped around a waist of the pet in a wearing state. The main body portion includes: a main-body first end which forms one end in the waistline direction; a main-body second end which forms the other end in the waistline direction, and arranged an outside of the disposable diaper in the wearing state; a main-body third end which forms one end in the cross direction, and arranged on a head-side of the pet in the wearing state; a main-body fourth end which forms the other end in the cross direction, and arranged on a crotch-side of the pet in the wearing state; and a mark portion being designed visible from a side of the backsheet of the disposable diaper for pet, and to indicate a position of the main-body second end in the wearing state. The mark portion includes: a head-side mark portion residing on a side of the main-body third end of a center in the cross direction of the main body portion; and a crotch-side mark portion residing on a side of the main-body fourth end of the center in the cross direction of the main body. The head-side mark portion being positioned inside or outside, in the waistline direction, of the crotch-side mark portion.

### BRIEF DESCRIPTION OF DRAWINGS

[Fig. 1] Fig. 1 is a plan view in which a disposable diaper according to a first embodiment of the present invention is seen from a skin contact surface side.
[Fig. 2] Fig. 2 is a plan view illustrating the disposable diaper for pet of the first embodiment, when viewed from the non-skin contact side.
[Fig. 3] Fig. 3 is a schematic cross sectional view of the disposable diaper for pet taken along the line A-A in Fig. 1.
[Fig. 4] Fig. 4 is a drawing schematically illustrating a wearing state of the disposable diaper for pet.
[Fig. 5] Fig. 5 is a plan view illustrating the disposable diaper for pet of the second embodiment, when viewed from the skin contact side.
[Fig. 6] Fig. 6 is a drawing schematically illustrating a wearing state of the disposable diaper for pet of the second embodiment.
[Fig. 7] Fig. 7 is a plan view illustrating the disposable diaper for pet of modified examples 1 to 3, when viewed from the non-skin contact side.
[Fig. 8] Fig. 8 is a plan view illustrating the disposable diaper for pet of modified examples 4 to 6, when viewed from the non-skin contact side.
[Fig. 9] Fig. 9 is a plan view illustrating the disposable diaper for pet of modified examples 7 to 9, when viewed from the non-skin contact side.
[Fig. 10] Fig. 10 is a plan view illustrating the disposable diaper for pet of modified examples 10 to 12, when viewed from the non-skin contact side.

### (1) Outline of Embodiment

According to the present specification and the accompanying drawings, at least the following matters will be disclosed.

A disposable diaper for pet according to one embodiment includes : a waistline direction disposed along a waistline direction of a pet; a cross direction orthogonal to the waistline direction; a main body portion having a topsheet, a backsheet, and an absorbent core arranged between the topsheet and the backsheet; and a binding portion that binds opposing parts of the main body portion when wrapped around a waist of the pet in a wearing state. The main body portion includes: a main-body first end which forms one end in the waistline direction; a main-body second end which forms the other end in the waistline direction, and arranged an outside of the disposable diaper in the wearing state; a main-body third end which forms one end in the cross direction, and arranged on a head-side of the pet in the wearing state; a main-body fourth end which forms the other end in the cross direction, and arranged on a crotch-side of the pet in the wearing state; and a mark portion being designed visible from a side of the backsheet of the disposable diaper for pet, and to indicate a position of the main-body second end in the wearing state. The mark portion includes: a head-side mark portion residing on a side of the main-body third end of a center in the cross direction of the main body portion; and a crotch-side mark portion residing on a side of the main-body fourth end of the center in the cross direction of the main body. The head-side mark portion being positioned inside, in the waistline direction, of the crotch-side mark portion. According to this embodiment, the user can align the main-body second end in the wearing state, referring to the mark portion. More specifically, the user places the main body portion around the waist of pet while aligning the waistline direction of the main body portion along the waist, and binds parts of the main body portion by the binding portion. Some sort of pet may have the head-side size shorter than the crotch-side size. With the head-side mark portion residing inside, in the waistline direction, of the crotch-side mark portion, and as a result of placement, by the user, of the main-body second end along the head-side mark portion and the crotch-side mark portion, the disposable diaper can be placed so as to make the head-side size shorter than the crotch-side size. The disposable diaper can therefore be placed conforming to the waist shape of pet, and can improve the fitting quality.

A disposable diaper for pet according to another embodiment include : a waistline direction disposed along a waistline direction of a pet; a cross direction orthogonal to the waistline direction; a main body portion having a topsheet, a backsheet, and an absorbent core arranged between the topsheet and the backsheet; and a binding portion that binds opposing parts of the main body portion when wrapped around a waist of the pet in a wearing state. The main body portion includes: a main-body first end which forms one end in the waistline direction; a main-body second end which forms the other end in the waistline direction, and arranged an outside of the disposable diaper in the wearing state; a main-body third end which forms one end in the cross direction, and arranged on a head-side of the pet in the wearing state; a main-body fourth end which forms the other end in the cross direction, and arranged on a crotch-side of the pet in the wearing state; and a mark portion being designed visible from a side of the backsheet of the disposable diaper for pet, and to indicate a position of the main-body second end in the wearing state. The mark portion includes: a head-side mark portion residing on a side of the main-body third end of a center in the cross direction of the main body portion; and a crotch-side mark portion residing on a side of the main-body fourth end of the center in the cross direction of the main body. The head-side mark portion being positioned outside, in the waistline direction, of the crotch-side mark portion. According to this embodiment, the user can align the main-body second end in the wearing state, referring to the mark portion. More specifically, the user places the main body portion around the waist of pet while aligning the waistline direction of the main body portion along the waist, and binds parts of the main body portion by the binding portion. Some sort of pet may have the head-side size longer than the crotch-side size. With the head-side mark portion positioned outside, in the waistline direction, of the crotch-side mark portion, and as a result of placement, by the user, of the main-body second end along the head-side mark portion and the crotch-side mark portion, the disposable diaper can be placed so as to make the head-side size longer than the crotch-side size. The disposable diaper can therefore be placed conforming to the waist shape of pet, and can improve the fitting quality.

According to a preferred embodiment, the binding portion is arranged on a face of a backsheet side of the main-body first end, and bound to a face of a topsheet side of the main-body second end in the wearing state. At least either the head-side mark portion or crotch-side mark portion is arranged in a region adjacent to the binding portion. According to this embodiment, the user can easily recognize the mark portion when handling the binding portion, and will become more aware of presence of the mark portion. Hence, the disposable diaper can therefore be placed conforming to the waist shape of pet, referring to the mark portion.

According to a preferred embodiment, the binding portion is arranged on a face of a backsheet side of the main-body first end, and bound to a side of a topsheet side of the main-body second end in the wearing state. At least either the head-side mark portion or crotch-side mark portion is arranged in a region overlapping the binding portion. According to this embodiment, the user can easily recognize the mark portion when handling the binding portion, and can place the disposable diaper conforming to the waist shape of pet, referring to the mark portion.

According to a preferred embodiment, at least either the head-side mark portion or the crotch-side mark portion indicates a position of an outer edge in the waistline direction of the main-body second end in the wearing state. According to this embodiment, the user can intuitively recognize the position and angle of the outer edge in the waistline direction of the main-body second end in the wearing state. By aligning the outer edge in the waistline direction of the main-body second end to the mark portion, the user can place the disposable diaper at a proper position and angle.

According to a preferred embodiment, the binding portion is arranged on the main-body first end. The mark portion is formed of the binding portion. When binding the main-body second end to the binding portion for placement according to this embodiment, it is a general practice to align the main-body second end along the binding portion. By aligning the main-body second end along the binding portion, the user can place the disposable diaper conforming to the waist shape of pet unconsciously.

According to a preferred embodiment, the binding portion has a head-side binding portion that composes the head-side mark portion, and a crotch-side binding portion that composes the crotch-side mark portion. The head-side binding portion and the crotch-side binding portion are arranged in the waistline direction with a space in between. According to this embodiment, with the head-side binding portion and the crotch-side binding portion arranged with a space in between, the user can stepwise bind the main-body second end, as compared with a structure having the head-side binding portion and the crotch-side binding portion integrated therein. Hence, even women or children with small hands can easily place the disposable diaper just as they intended, and can improve the fitting quality.

According to a preferred embodiment, the head-side mark portion and the crotch-side mark portion are connected and extend in the cross direction, obliquely to the cross direction. With the head-side mark portion and the crotch-side mark portion connected, this embodiment enables sequential alignment of the main-body second end, and gradual binding of the main-body second end to the binding portion. Hence, even women or children with small hands can easily place the disposable diaper just as they intended, and can improve the fitting quality.

According to a preferred embodiment, the mark portion is formed of an outer edge on a side of the main-body first end of the absorbent core. This embodiment can also provide guidance to a placement position of the main-body second end, with an improved decorativeness given by the printed part.

According to a preferred embodiment, the mark portion may be formed of the outer edge on the main-body first end side of the absorbent core. According to this embodiment, the user will become easier to recognize a position of the outer edge of the absorbent core, in the process of alignment referring to the mark portion for placement. By recognizing the position of the outer edge of the absorbent core, the user can dispose the absorbent core so as not to cause leakage.

A method for manufacturing disposable diaper for pet, the disposable diaper for pet comprising the features of claim 1 is also claimed and includes a sheet feeding step of feeding a continuous sheet having sheet components arrayed; a marking step of giving a mark portion on the continuous sheet; and a core laminating step of laminating an absorbent core on the continuous sheet having the mark portion. The core laminating step being designed to detect a position of the mark portion on the continuous sheet, and to adjust a position of lamination of the absorbent core referring to the position of the mark portion. With present method for manufacturing, the positional relation between the mark portion and the absorbent core may be kept constant. Hence, the disposable diaper when placed referring to the mark portion will have the absorbent core whose placement position is prevented from being misaligned one by one. With suppression of shift of the mark portion over the whole range of the disposable diaper, alignment referring to such mark portion will be enabled with improved accuracy.

### (2) Overall Structure of Disposable diaper for pet

A disposable diaper for pet according to an embodiment will be described below by referring to the accompanying drawings. In the drawings, the same or similar parts are indicated by the same or similar reference signs. The drawings are illustrated schematically, and dimensional ratio and other variables differ from those of actual measurements. The actual measurements or the like, therefore, should be determined by referring to the following description. The drawings may include different relationships or ratios of measurements.

In this patent specification, "pet" widely encompasses vertebrate and invertebrate animals, and typically include pet animals such as cat, dog, rabbit and hamster. The disposable diaper in this embodiment is a disposable diaper for dog. In a modified example, the disposable diaper may be the one for cat.

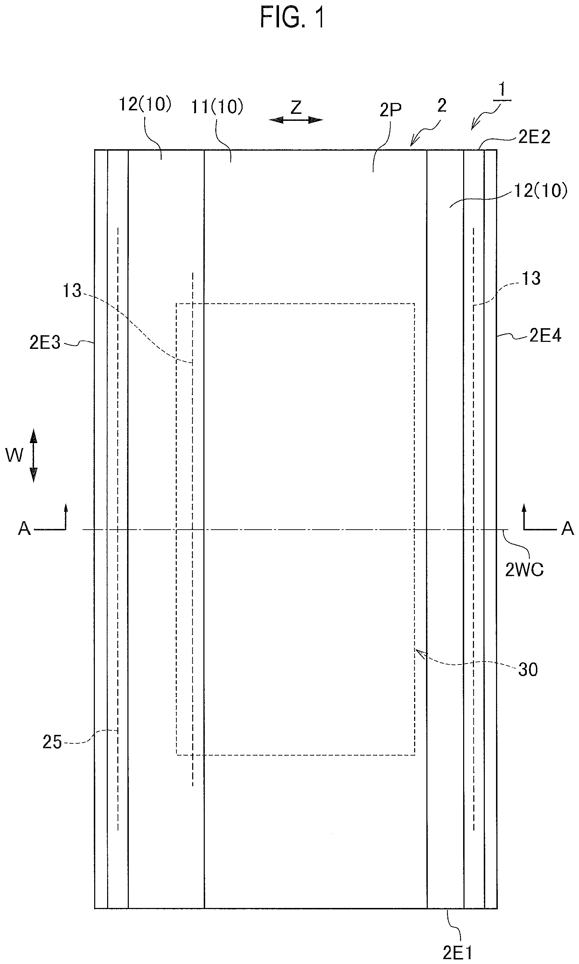

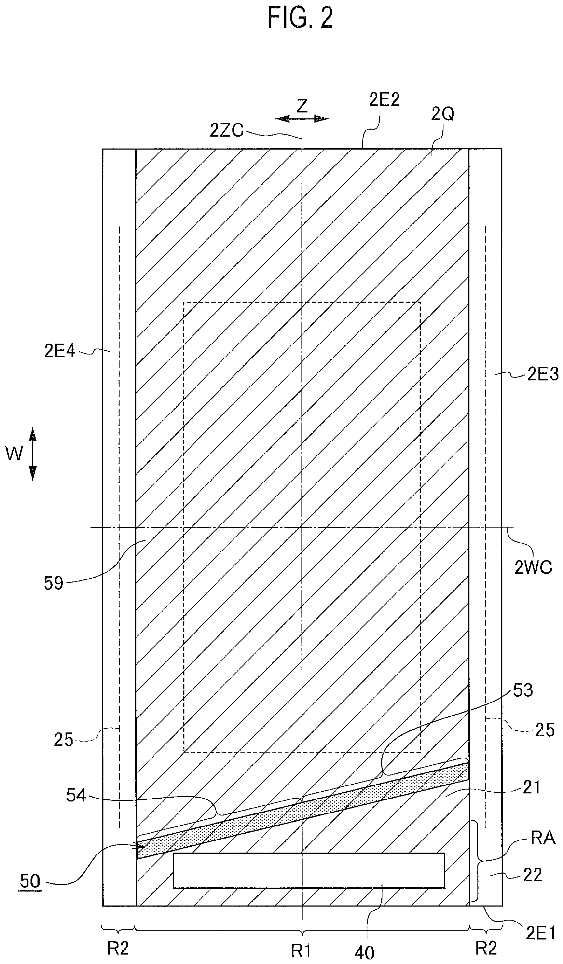

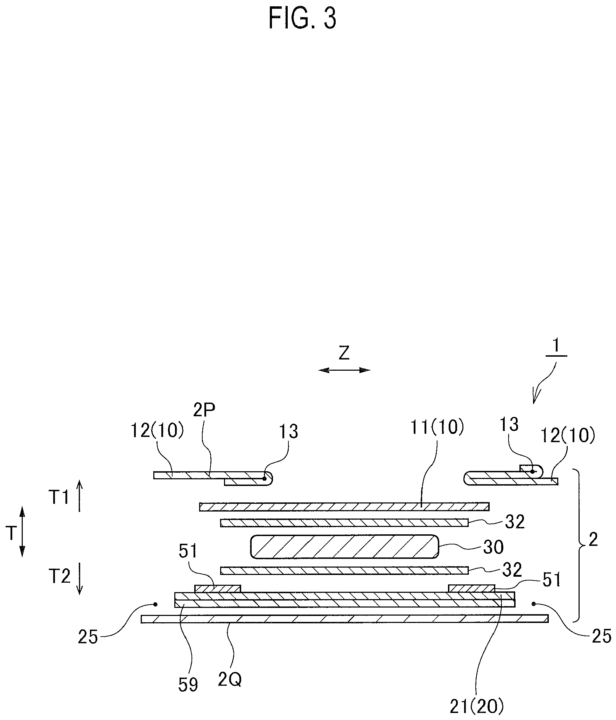

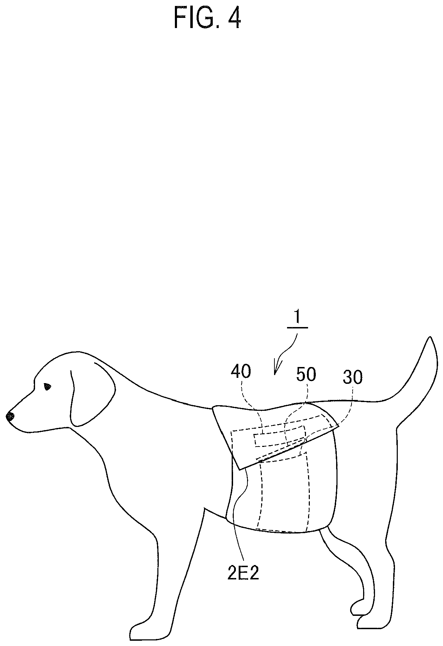

Fig. 1 is a plan view illustrating the disposable diaper for pet of the first embodiment, when viewed from the skin contact side. Fig. 2 is a plan view illustrating the disposable diaper for pet of the first embodiment, when viewed from the non-skin contact side. Fig. 3 is a schematic cross sectional view of the disposable diaper for pet taken along the line A-A in Fig. 1. Figs. 1 and 2 illustrate a disposable diaper for pet 1, extended until any wrinkle will disappear. The individual members, although illustrated in the cross sectional view of Fig. 3 as if they are apart from each other in the thickness direction T for the convenience of explanation, are brought into close contact in the thickness direction T in the actual product. Fig. 4 is a drawing schematically illustrating a wearing state of the disposable diaper for pet.

The disposable diaper for pet has waistline direction W laid in the waistline direction of the pet, cross direction Z laid orthogonal to the waistline direction W, and the thickness direction T laid orthogonal to the waistline direction W and to the cross direction Z. The cross direction Z is laid in the longitudinal direction of the pet, that is, the direction connecting the crotch-side with the far side of the crotch. The thickness direction T is laid towards the top face side T1 faced to the pet in the wearing state, and towards the back face side T2 faced outside in the wearing state. As illustrated in Fig. 4, the disposable diaper for pet of the first embodiment is placed so as to wrap around the waist of the pet, rather than being placed so as to cover the pet from the belly through the crotch to the back. Hence the length in the waistline direction W of the disposable diaper for pet 1 is longer than the length in the cross direction Z of the disposable diaper for pet 1.

The disposable diaper for pet 1 has a main body portion 2 and a binding portion 40. The main body portion 2 may have a main-body first end 2E1 which forms one end in the waistline direction W, a main-body second end 2E2 which forms the other end in the waistline direction W, a main-body third end 2E3 which forms one end in the cross direction Z, and a main-body fourth end 2E4 which forms the other end in the cross direction Z. The main-body first end 2E1, when placed around the waist of pet in the wearing state (see Fig. 4) is covered with the main-body second end 2E2, and positioned inside (pet side) of the disposable diaper. The main-body second end 2E2 is opposed to the main-body first end 2E1, and is disposed on the outside of the disposable diaper in the wearing state. The main-body third end 2E3 is disposed on the head-side of the pet in the wearing state. The main-body fourth end 2E4 is opposed to the main-body third end 2E3, and is disposed on the crotch side (hip side) of the pet in the wearing state.

The main body portion 2 has at least a top sheet 10, a backsheet 20, and an absorbent core 30. The topsheet 10 is arranged on an inner face 2P of the main body portion 2, which is brought into contact with the pet. The topsheet 10 has liquid permeability that allows body fluid to permeate towards the absorbent core 30. The topsheet 10 may have a center sheet 11 arranged at the center in the cross direction Z so as to cover the absorbent core 30, and side sheets 12 individually covering both side part in the cross direction Z of the center sheet 11. The topsheet 10 may have thereon a printed part. The printed part may form a mark portion 50 described later.

The side sheets 12 may be folded as illustrated in Fig. 3. More specifically, the side sheet 12 on a main-body third end 2E3 side (left side in Fig. 1) is folded back at the inner end of the side sheet 12 towards the back face side T2. Meanwhile, the side sheet 12 on a main-body fourth end 2E4 side (right side in Fig. 1) is folded back at the inner end of the side sheet 12 towards the top face side T1, and the thus folded part is again folded back towards the top face side T1. A side elastic member 13 while being stretched in the waistline direction W is arranged in between folded parts of the side sheets 12. The side elastic member 13 may form leakproof gathers that rise up towards the pet. In the wearing state, one end side in the cross direction Z may be arranged on the front side of the pet, and the other end side in the cross direction Z may be arranged on the back side the pet in the wearing state. The side sheet on one end side in the cross direction Z and the side sheet on the other end side in the cross direction, although being asymmetric in this embodiment, may be symmetric in a modified example. The end in the context of this invention is defined as a part that occupies a certain range including the edge.

The absorbent core 30 is arranged between the topsheet 10 and the backsheet 20. The absorbent core 30 is made of a stack of pulp or other absorption materials. The absorbent core 30 may have a core wrap 32 that covers the absorbent core 30. The absorbent core 30 is arranged, in the waistline direction W, inside of both edges 2WE of the main body portion 2. That is, the length in the waistline direction W of the absorbent core 30 is shorter than the length in the waistline direction W of the main body portion 2. The absorbent core 30 is arranged at the center in the waistline direction W of the main body portion 2, but is not arranged outside in the waistline direction W of the main body portion 2. The absorbent core 30 may be arranged, in the cross direction Z, inside of both edges 2ZE of the main body portion 2. That is, the length in the cross direction Z of the absorbent core 30 may be shorter than the length in the cross direction Z of the main body portion 2. The absorbent core 30 may be arranged at the center in the cross direction Z of the main body portion 2, but is not necessarily arranged outside in the cross direction Z of the main body portion 2. The absorbent core 30 and the core wrap 32 may have arranged thereto a printed part. The printed part may form a mark portion 50.

The backsheet 20 is arranged on an outer face 2Q of the main body portion 2, which appears on the outside in the wearing state. The backsheet 20 may have a non-liquid permeable back film 21 and a nonwoven back fabric 22 arranged on the outer side of the back film 21. In a modified example, the backsheet may have a non-liquid permeable back film and a nonwoven back fabric arranged on the inner face side of the back film 21. The back film 21 may have, on a face thereof on the top face side T1, an indicator that causes a coloring reaction upon contact with body fluid, and a printed part, meanwhile the back film 21 may have, on a face thereof on the back face side T2, a printed part. At least one of the indicator or the printed part may form the mark portion 50. That is, the length in the cross direction Z of the back film 21 may be shorter than the length in the cross direction Z of the nonwoven back fabric 22. That is, the nonwoven back fabric 22 may extend on both sides in the cross direction Z out from the back film 21. In an embodiment where the printed part is arranged entirely over the back film 21, an area where the back film 21 is arranged may form a printed area R1, and a non-printed area R2 free of printing may be arranged on both sides in the cross direction Z of the back film 21.

The main body portion 2 may have, on both ends thereof in the cross direction Z, waistline gathers that stretch and contract in the waistline direction W. The waistline gathers are positioned outside in the cross direction Z of the absorbent core 30, and may be composed of the topsheet 10, the backsheet 20, and a waistline elastic member 25. The waistline elastic member 25 may be arranged between the topsheet 10 and the backsheet 20, and may be fixed, while being stretched in the waistline direction W, to the topsheet 10 and the backsheet 20. The waistline elastic member 25 and the aforementioned side elastic member 13 compose the elastic member in this invention.

The binding portion 40, in the wearing state, binds the opposing parts of the main body portion 2. More specifically, the binding portion 40 is a binding means that binds the outer face 2Q of the main-body first end 2E1 to the inner face 2P of the main-body second end 2E2. The binding portion 40 in this embodiment is arranged on the main-body first end 2E1 of the main body portion 2. In more detail, the binding portion 40 is a mechanical fastener arranged on the outer face of the main-body first end 2E1. The binding portion 40 is made so as to be bound on the topsheet 10 of the main-body second end 2E2 of the main body portion 2. The disposable diaper for pet 1 may be placed on the pet, by placing it so as to cover the binding portion 40 with the main-body second end 2E2, by which the binding portion 40 is bound on the topsheet 10 of the main-body second end 2E2, and can keep the wearing state of the disposable diaper for pet. In a modified example, the binding portion 40 may be arranged on the inner face 2P of the main-body second end 2E2, so as to be bound on the backsheet 20 of the main-body first end 2E1. That is, in the modified example, the binding portion 40 may be arranged on the inner face 2P of the main body portion 2, so as to be bound on the backsheet of the main body portion 2.

Next, the mark portion 50 will be explained. The mark portion 50 provides guidance to a placement position of the disposable diaper for pet 1. The mark portion 50 is made visually recognizable from the backsheet side (outer face side) of the disposable diaper for pet 1. The backsheet side of the disposable diaper for pet, when placed, appears on the outer side and is visually recognizable after placing the disposable diaper to the pet, enabling positional adjustment not only in the process of placement, but also after placement. Even if the disposable diaper should turn in the waistline direction to cause displacement during wearing, such displacement is recognizable referring to the mark portion, enabling repositioning to the suitable position.

Now "visually recognizable" in the context of this invention means that a subject with healthy vision in both eyes (20/20 vision or higher) can recognize an object from approximately 30 to 50 cm away, in a bright (approximately 500 to 750 lx) room with daylight illumination (color temperature ranging from 4600 to 5400 K). The mark portion 50 may have at least one of the first mark portion 51 or the second mark portion 52.

The mark portion 50 indicates a position of the main-body second end 2E2 in the wearing state. The mark portion 50 in the wearing state is arranged in an area that overlaps the main-body second end 2E2, so that the user can dispose the main-body second end 2E2 so as to make it overlap the mark portion 50. In another embodiment, the mark portion 50 may be an arrow that points a position of the main-body second end 2E2 in the wearing state, and the user may dispose the main-body second end 2E2 at the position pointed by the mark portion 50. The mark portion 50 in this embodiment indicates a position of the edge of the main body portion 2 in the wearing state in the main-body second end 2E2.

The mark portion 50 has a head-side mark portion 53 that resides on the main-body third end 2E3 side of the center 2ZC in the cross direction Z of the main body portion 2, and a crotch-side mark portion 54 that resides on the main-body fourth end 2E4 side of the center 2ZC in the cross direction Z of the main body portion 2. The head-side mark portion 53 and the crotch-side mark portion 54 may separate from each other, or may be integrated. In a mode where the head-side mark portion 53 and the crotch-side mark portion 54 are integrated, the boundary between the head-side mark portion 53 and the crotch-side mark portion 54 may lie on the center 2ZC in the cross direction Z of the main body portion 2. The head-side mark portion 53 resides inside, in the waistline direction W, of the crotch-side mark portion 54. The inside in the waistline direction W means the center 2WC side in the waistline direction W of the main body portion 2. Hence, at least a part of the head-side mark portion 53 resides on the center 2WC side in the waistline direction W of the main body portion 2, than the crotch-side mark portion 54 does.

The waistline direction W of the main body portion 2 is laid around the waistline direction of the pet, meanwhile the cross direction Z of the main body portion 2 is disposed along the front-back direction that extends from the head side to the crotch side. The waist size of pet varies in the front-back direction, and some pet has a waist size shorter on the head side, than on the crotch side. Hence, a disposable diaper for pet having a rectangular main body portion 2 in plan view has occasionally produced a gap between the waist of pet and the disposable diaper, degrading the fitting quality. Waist size of pet varies depending on species of pet, and also changes depending on seasons due to molting. Hence the user may feel it difficult to place the disposable diaper well conforming to the shape of pet body.

In contrast, according to this embodiment, the user can place the disposable diaper so as to make the head-side size shorter than the crotch-side size, by aligning a position in the wearing state of the main-body second end 2E2, referring to the mark portion 50. More specifically, as illustrated in Fig. 4, the user places the main body portion 2 around the waist of pet while aligning the waistline direction W of the main body portion 2 along the waist, and binds parts of the main body portion 2 with the binding portion 40. With the head-side mark portion 53 residing inside, in the waistline direction W, of the crotch-side mark portion 54, and as a result of placement of the main-body second end 2E2 along the head-side mark portion 53 and the crotch-side mark portion 54, the disposable diaper can be placed so as to make the head-side size shorter than the crotch-side size. Placement well conforming to the shape of the waist of pet can improve the fitting quality.

The binding portion 40 in this embodiment is arranged on a face on the backsheet side of the main-body first end 2E1, and is formed so as to be bound with a face on the topsheet side of the main-body second end 2E2 in the wearing state. At least either the head-side mark portion 53 or the crotch-side mark portion 54 may be arranged in an adjoining area RA that adjoins the binding portion 40. The adjoining area RA is a region around the binding portion 40 in plan view, and may preferably be an area within 20 mm from the binding portion 40. Fig. 2 illustrates a range in the waistline direction of the adjoining area RA. The user, handling the disposable diaper for pet 1 for placement, can recognize not only the binding portion 40, but also the adjoining area RA around it. Hence the user will become more aware of presence of the mark portion 50, and can place the disposable diaper conforming to the waist shape of pet, referring to the mark portion 50.

At least either the head-side mark portion 53 or the crotch-side mark portion 54 may point the outer edge in the waistline direction W of the main-body second end 2E2 in the wearing state. The user can intuitively recognize the position and angle of the outer edge in the waistline direction W of the main-body second end 2E2 in the wearing state. By aligning the outer edge in the waistline direction W of the main-body second end 2E2 to the mark portion 50, the user can place the disposable diaper at a proper position and angle.

The head-side mark portion 53 and the crotch-side mark portion 54 may be connected, and may extend in the cross direction Z, obliquely to the cross direction Z. With the head-side mark portion 53 and the crotch-side mark portion 54 connected, the main-body second end 2E2 may be aligned sequentially. The user can therefore recognize the mark portion 50, and can gradually bind the main-body second end 2E2 with the binding portion 40, enabling easy placement at a proper position.

The mark portion 50 may be a printed part arranged on the backsheet 20. The printed part can take part in guidance to a placement position of the main-body second end 2E2, while enhancing the decorativeness. The back film 21 of this embodiment has the printed part arranged over the entire range of the outer face. The printed part may form the mark portion 50, as well as the decoration part 59. As illustrated in Fig. 2, the back film in this embodiment has, on the outer face thereof, a printed part that forms the mark portion 50, and a printed part that forms a decoration part 59. The decoration part 59 is a part having design other than the mark portion, and has not only picture or pattern, but also background color solely represented by color. In a modified example, the mark portion may be an indicator arranged on the backsheet 20 or the core wrap 32.

In another embodiment, the mark portion 50 may be a boundary mark portion. More specifically, the printed part may have a first decoration part, and a second decoration part designed differently from the first decoration part, and the boundary between the first decoration part and the second decoration part may form a boundary mark portion. This embodiment enables alignment referring to the mark portion 50, while enjoying improved decorativeness given by two designs of the first decoration part and the second decoration part.

The backsheet 20 may have, as illustrated in Fig. 2, the printed area R1 having printed therein a design including the mark portion 50, and the non-printed area R2 free of printing. The non-printed areas R2 may be arranged at least on both ends in the cross direction Z of the backsheet 20. The user can recognize both ends in the cross direction Z of the main body portion 2, referring to a boundary between the non-printed area R2 and the printed area R1. Provision of the non-printed area R2 makes the printed area R1 more distinctive, making the mark portion 50 more easily recognizable in the printed area R1. The boundary between the non-printed area R2 and the printed area R1 may extend in the waistline direction W, and may reside outside in the cross direction Z of the absorbent core 30. That is, the non-printed area R2 may be arranged only on the outer side in the cross direction Z of the absorbent core 30. The user can recognize an area where the absorbent core 30 is not arranged, referring to the non-printed area R2, and can thereby position the absorbent core 30 so as not to cause leakage.

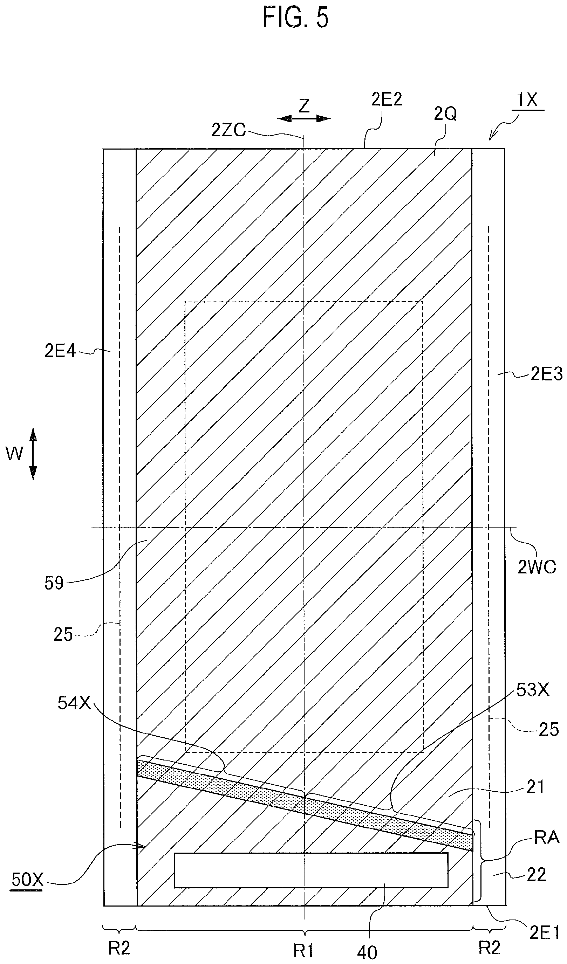

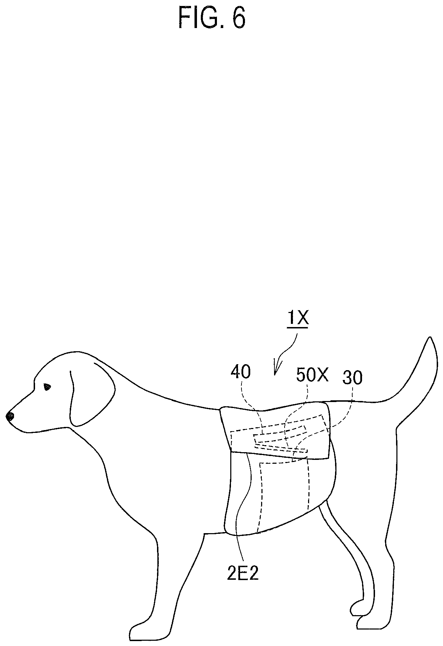

Next, a disposable diaper for pet 1X of the second embodiment will be explained, referring to Figs. 5 and 6. Fig. 5 is a plan view illustrating the disposable diaper for pet of the second embodiment, when viewed from the non-skin contact side. Fig. 6 is a drawing schematically illustrating the wearing state of the disposable diaper for pet of the second embodiment. Note that all structures same as those in the aforementioned embodiment will be given the same reference signs to avoid redundant explanation. The mark portion 50X of the disposable diaper for pet 1X of the second embodiment has a head-side mark portion 53X and a crotch-side mark portion 54X. The head-side mark portion 53X resides outside, in the waistline direction W, of the crotch-side mark portion 54X. Hence, at least a part of the crotch-side mark portion 54X resides on the center 2WC side in the waistline direction W of the main body portion 2, than the head-side mark portion 53X does.

The waistline direction W of the main body portion 2 is laid around the waistline direction of the pet, meanwhile the cross direction Z of the main body portion 2 is disposed along the front-back direction that extends from the head side to the crotch side. The waist size of the pet varies in the front-back direction, and some pet has a waist size longer on the head side, than on the crotch side. Hence, a disposable diaper for pet having a rectangular main body portion 2 in plan view has occasionally produced a gap between the waist of pet and the disposable diaper, degrading the fitting quality. Waist size of pet varies depending on species of pet, and also changes depending on seasons due to molting. Hence the user may feel it difficult to place the disposable diaper well conforming to the shape of pet body.

In contrast, according to this embodiment, the user can place the disposable diaper so as to make the head-side size longer than the crotch-side size, by aligning a position in the wearing state of the main-body second end 2E2, referring to the mark portion 50X. More specifically, as illustrated in Fig. 6, the user places the main body portion 2 around the waist of pet while aligning the waistline direction W of the main body portion 2 along the waist, and binds parts of the main body portion 2 with the binding portion 40. With the head-side mark portion 53X residing outside, in the waistline direction W, of the crotch-side mark portion 54X, and as a result of placement of the main-body second end 2E2 along the head-side mark portion 53X and the crotch-side mark portion 54X, the disposable diaper can be placed so as to make the head-side size longer than the crotch-side size. Placement well conforming to the shape of the waist of pet can improve the fitting quality.

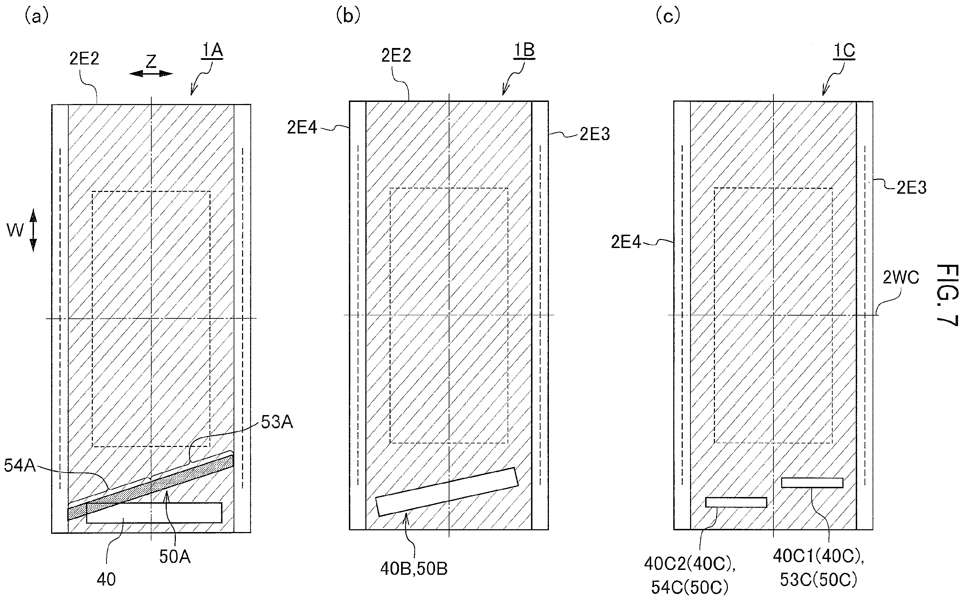

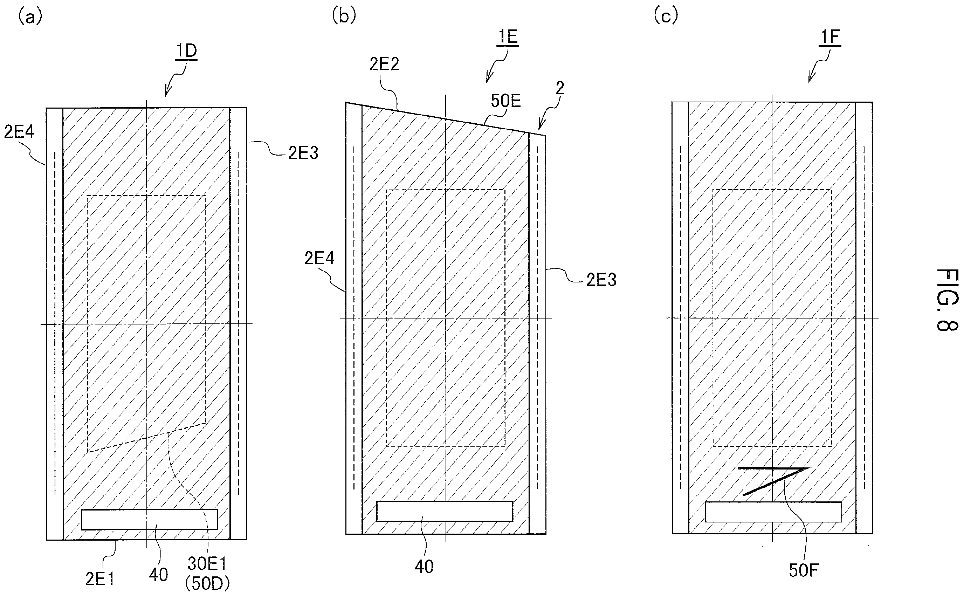

Next, a disposable diaper for pet of modified examples will be explained. Note that all structures same as those in the aforementioned embodiment will be given the same reference signs to avoid redundant explanation. Figs. 7 and 8 are plan views illustrating modified examples of the disposable diaper for pet of the first embodiment, viewed from the non-skin contact side of the disposable diaper for pet.

Fig. 7(a) illustrates a disposable diaper for pet 1A of Modified Example 1. The disposable diaper for pet 1A of modified example 1 has a mark portion 50A in which at least either a head-side mark portion 53A or a crotch-side mark portion 54A is arranged in an area that overlaps the binding portion 40. More specifically, the crotch-side mark portion 54A is arranged in the area that overlaps the binding portion 40. Note that at least a part of the crotch-side mark portion 54A may overlap the binding portion 40, or at least a part of the head-side mark portion 53A may overlap the binding portion 40. The user can easily recognize the mark portion 50A when handling the binding portion. The disposable diaper for pet 1A of modified example 1 may easily be placed conforming to the waist shape of pet, referring to the mark portion 50A.

Fig. 7(b) illustrates a disposable diaper for pet 1B of modified example 2. A mark portion 50B of a disposable diaper for pet 1B of modified example 2 is formed of a binding portion 40B. The edge in the waistline direction of the binding portion 40B in modified example 2 inclines towards the inside in the waistline direction, when viewed from the main-body fourth end 2E4 towards the main-body third end 2E3. The binding portion 40B has a rectangular shape in plan view. The shape in plan view of the binding portion 40B may be rectangle (square) or parallelogram. When binding the main-body second end 2E2 to the binding portion 40B for placement, it is a general practice to align the main-body second end 2E2 along the binding portion 40B. The user can place the disposable diaper for pet 1B of modified example 2 so as to unconsciously shrink it towards the head side, by aligning the main-body second end 2E2 along the binding portion 40B.

Fig. 7(c) illustrates a disposable diaper for pet 1C of modified example 3. The disposable diaper for pet 1C of modified example 3 has a mark portion 50C that is formed of a binding portion 40C, and the binding portion 40C has a head-side binding portion 40C1 composing a head-side mark portion 53C and a crotch-side binding portion 40C2 composing a crotch-side mark portion 54C. The head-side binding portion 40C1 and the crotch-side binding portion 40C2 are arranged in plan view with a space in between. The head-side binding portion 40C1 and the crotch-side binding portion 40C2 may only be apart, allowing arrangement with a space in between in the waistline direction W, or arrangement with a space in between in the cross direction Z. Each of the head-side binding portion 40C1 and the crotch-side binding portion 40C2 of modified example 3 extends in the cross direction Z, and the center in the waistline direction W of the head-side binding portion 40C1 resides on the center 2WC side in the waistline direction W of the main body portion 2, than the center in the waistline direction W of the crotch-side binding portion 40C2. With the head-side binding portion 40C1 and the crotch-side binding portion 40C2 arranged with a space in between, the user can stepwise bind the main-body second end 2E2, as compared with a structure having the head-side binding portion and the crotch-side binding portion integrated therein. Hence, one binding portion may be temporarily bound with the other binding portion being regularly bound, and then the one binding portion may be unbound and then re-bound. According to this mode of binding, the disposable diaper may easily be placed in a well placed state on the body with improved fitting quality, as compared with the mode where the binding portion is bound in a single operation. This mode of binding also allows women or children with small hands to easily place the disposable diaper just as they intended, and can improve the fitting quality.

Fig. 8(a) illustrates a disposable diaper for pet ID of a modified example 4. A mark portion 50D of the disposable diaper for pet ID of modified example 4 is formed of an outer edge 30E1 on the main-body first end 2E1 side of the absorbent core 30. The outer edge 30E1 on the main-body first end 2E1 side of the absorbent core 30 is an outer edge in the waistline direction of the absorbent core 30, and inclines towards the inside in the waistline direction W, when viewed from the main-body fourth end 2E4 towards the main-body third end 2E3. According to the disposable diaper for pet ID of modified example 4, the user will become easier to recognize a position of the outer edge of the absorbent core 30, in the process of alignment referring to the mark portion 50D for placement. By recognizing the position of the outer edge of the absorbent core, the user can dispose the absorbent core 30 so as not to cause leakage.

Fig. 8(b) illustrates a disposable diaper for pet IE of modified example 5. A mark portion 50E of the disposable diaper for pet IE of Modified Example 5 is formed of an outer edge on the main-body second end 2E2 side of the main body portion 2. The outer edge on the main-body second end 2E2 side of the main body portion 2 inclines in the cross direction Z and the waistline direction W, and inclines towards the inside in the waistline direction W, when viewed from the main-body fourth end 2E4 towards the main-body third end 2E3. According to the disposable diaper for pet IE of Modified Example 5, the user can intuitively recognize the position and angle of the outer edge in the waistline direction of the main-body second end in the wearing state, enabling placement at a proper position and angle.

Fig. 8(c) illustrates a disposable diaper for pet IF of Modified Example 6. A mark portion 50F of the disposable diaper for pet IF of Modified Example 6 indicates an angle formed between the outer edge in the waistline direction W of the main-body first end 2E1 in the wearing state, and the outer edge in the waistline direction W of the main-body second end 2E2. According to the disposable diaper for pet IF of Modified Example 6, the user can intuitively recognize the position and angle of the outer edge in the waistline direction of the main body in the wearing state, enabling placement at a proper position and angle.

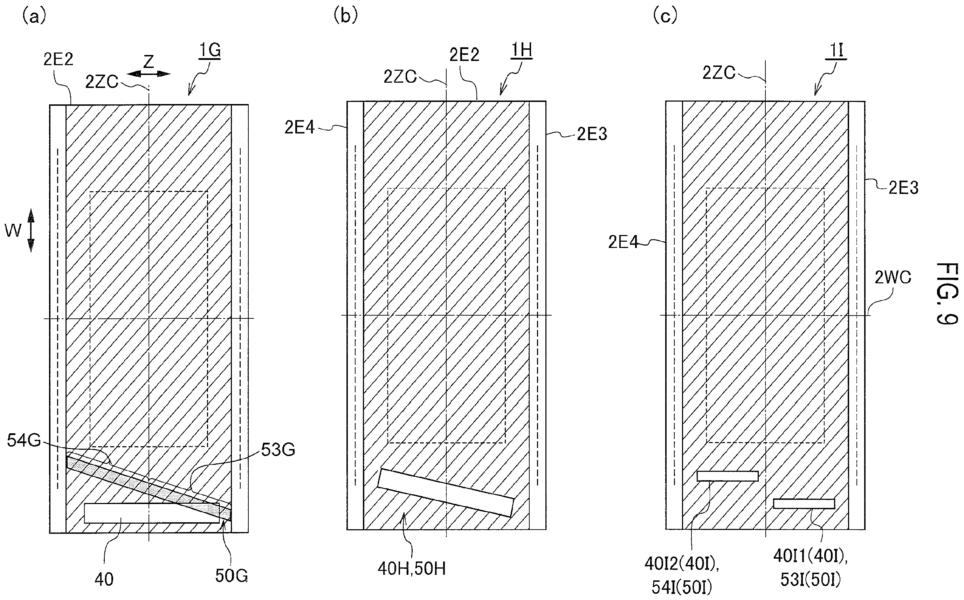

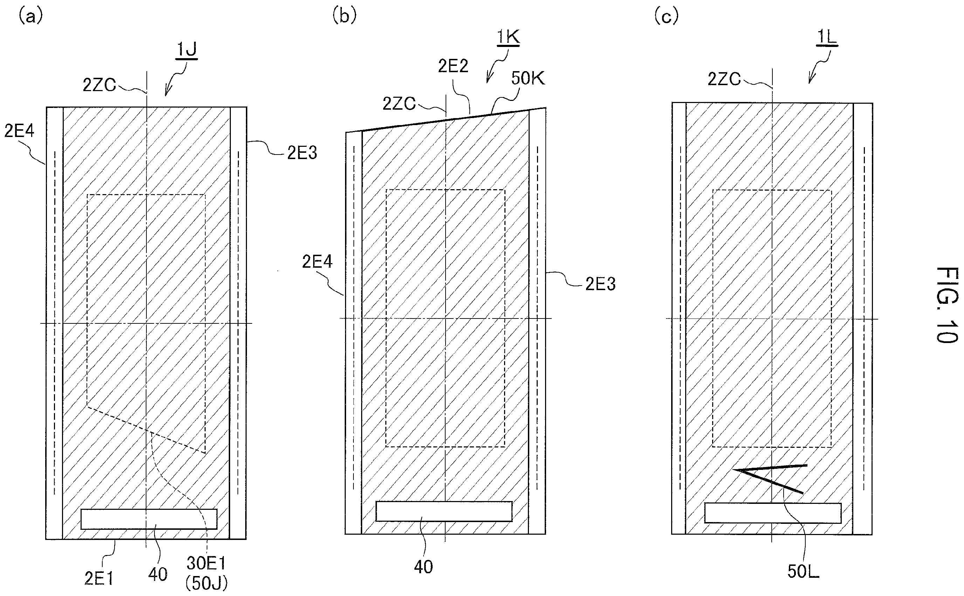

Figs. 9 and 10 are plan views illustrating modified examples of the disposable diaper for pet of the second embodiment, viewed from the non-skin contact side of the disposable diaper for pet. Fig. 9(a) illustrates a disposable diaper for pet 1G of Modified Example 7. A mark portion 50G of the disposable diaper for pet 1G of Modified Example 7, and the mark portion 50A of the disposable diaper for pet 1A of Modified Example 1 are axisymmetric about the center 2ZC in the cross direction Z of the main body portion 2. In the disposable diaper for pet 1G of Modified Example 7, at least either of a head-side mark portion 53G or a crotch-side mark portion 54G is arranged in an area that overlaps the binding portion 40. The head-side mark portion 53G resides outside, in the waistline direction, of the crotch-side mark portion 54G.

Fig. 9(b) illustrates a disposable diaper for pet 1H of Modified Example 8. A mark portion 50H of the disposable diaper for pet 1H of Modified Example 8, and the mark portion 50B of the disposable diaper for pet 1B of Modified Example 2 are axisymmetric about the center 2ZC in the cross direction Z of the main body portion 2. The mark portion 50H of the disposable diaper for pet 1H of Modified Example 8 is formed of a binding portion 40H. The edge in the waistline direction of the binding portion 40H in Modified Example 8 inclines towards the inside in the waistline direction, when viewed from the main-body third end 2E3 towards the main-body fourth end 2E4.

Fig. 9(c) illustrates a disposable diaper for pet 1I of Modified Example 9. A mark portion 501 of the disposable diaper for pet 1I of Modified Example 9, and the mark portion 50C of the disposable diaper for pet 1C of Modified Example 3 are axisymmetric about the center 2ZC in the cross direction Z of the main body portion 2. The disposable diaper for pet 1I of modified example 9 has a mark portion 501 that is formed of a binding portion 401, and the binding portion 401 has a head-side binding portion 4011 composing a head-side mark portion 531 and a crotch-side binding portion 4012 composing a crotch-side mark portion 54I. The head-side binding portion 4011 and the crotch-side binding portion 4012 are arranged in plan view with a space in between. Each of the head-side binding portion 4011 and the crotch-side binding portion 4012 of modified example 9 extends in the cross direction Z, and the center in the waistline direction W of the head-side binding portion 4011 resides more outward in the waistline direction W of the main body portion 2, than the center in the waistline direction W of the crotch-side binding portion 40I2.

Fig. 10(a) illustrates a disposable diaper for pet 1J of Modified Example 10. A mark portion 50J of the disposable diaper for pet 1J of Modified Example 10, and the mark portion 50D of the disposable diaper for pet ID of Modified Example 4 are axisymmetric about the center 2ZC in the cross direction Z of the main body portion 2. The mark portion 50J of the disposable diaper for pet 1J of Modified Example 10 is formed of the outer edge 30E1 on the main-body first end 2E1 side of the absorbent core 30. The outer edge 30E1 on the main-body first end 2E1 side of the absorbent core 30 is an outer edge in the waistline direction of the absorbent core 30, and inclines towards the inside in the waistline direction W, when viewed from the main-body third end 2E3 towards the main-body fourth end 2E4.

Fig. 10(b) illustrates a disposable diaper for pet 1K of Modified Example 11. A mark portion 50K of the disposable diaper for pet 1K of Modified Example 11, and the mark portion 50E of the disposable diaper for pet 1E of Modified Example 5 are axisymmetric about the center 2ZC in the cross direction Z of the main body portion 2. The mark portion 50K of the disposable diaper for pet 1K of Modified Example 11 is formed of an outer edge on the main-body second end 2E2 side of the main body portion 2. The outer edge on the main-body second end 2E2 side of the main body portion 2 inclines in the cross direction Z and the waistline direction W, and inclines towards the inside in the waistline direction W, when viewed from the main-body third end 2E3 towards the main-body fourth end 2E4.

Fig. 10(c) illustrates a disposable diaper for pet 1L of Modified Example 12. A mark portion 50L of the disposable diaper for pet 1L of Modified Example 12, and the mark portion 50F of the disposable diaper for pet IF of Modified Example 6 are axisymmetric about the center 2ZC in the cross direction Z of the main body portion 2. The mark portion 50L of the disposable diaper for pet 1L of Modified Example 12 indicates an angle formed between the outer edge in the waistline direction W of the main-body first end 2E1 in the wearing state, and the outer edge in the waistline direction W of the main-body second end 2E2.

Next, an exemplary method for manufacturing the thus designed disposable diaper for pet will be explained. The method for manufacturing the disposable diaper for pet may include:
a sheet feeding step of feeding a continuous sheet having sheet components arrayed therein;
a marking step of giving a mark portion by printing on the continuous sheet to provide the mark portion 50; and
an absorbent core laminating step of laminating the absorbent core 30 on the continuous sheet having the mark portion 50.

The sheet feeding step, feeding the continuous sheet having sheet components arrayed therein, takes part in feeding of the continuous sheet in which sheet components, composing any one of the topsheet 10, backsheet and core wrap, are arrayed. The marking step takes part in giving the mark portion 50 on the continuous sheet. The mark portion 50 may be given by printing, or by embossing. The core laminating step takes part in detection of the mark portion 50 on the continuous sheet. The mark portion 50 may be detected by detecting a position thereof using an image capturing means such as camera. The core laminating step takes part in adjustment of a position of lamination of the absorbent core 30, referring to the position of the mark portion 50. Adjustment of the position of lamination of the absorbent core 30 means adjustment of the positional relation between the absorbent core 30 and the continuous sheet. More specifically, the positional relation between the continuous sheet and the absorbent core 30 is adjustable by controlling the rate of feeding of the continuous sheet. Alternatively, an adjustment mechanism may be arranged adjacent to the sides, in a cross direction orthogonal to the feeding direction, of the continuous sheet, and a position in the orthogonal direction of the continuous sheet may be adjusted using such adjustment mechanism. With such method for manufacturing, the positional relation between the mark portion 50 and the absorbent core 30 may be kept constant. Hence, the disposable diaper when placed referring to the mark portion 50 will have the absorbent core 30 whose placement position is prevented from being misaligned one by one. With suppression of shift of the mark portion 50 over the whole range of the disposable diaper, alignment referring to such mark portion 50 will be enabled with improved accuracy.

Next, a packaged article having a plurality of disposable diapers for pet enclosed therein will be explained. The packaged article includes a plurality of disposable diapers for pet, and a wrapper for enclosing the plurality of disposable diapers for pet. The plurality of disposable diapers for pet includes a first disposable diaper having the first decoration part printed on the backsheet, and a second disposable diaper having the second decoration part designed differently from the first decoration part printed on the backsheet. The first decoration part and the second decoration part may only have design different from each other, and may be different from the first decoration part and the second decoration part in the second embodiment. The disposable diaper in this embodiment has the first decoration part or the second decoration part, printed on the back film of the backsheet. With such first disposable diaper having the first decoration part and the mark portion, and the second disposable diaper having the second decoration part and the mark portion, this embodiment will have enhanced decorativeness, thereby can attract attention of the user, and can improve recognizability of the mark portion. The first decoration part and the second decoration part are parts having a design other the mark portion, and may have not only picture or pattern, but also background color solely represented by color.

### [INDUSTRIAL APPLICABILITY]

According to the present invention, it is possible to provide a disposable diaper for pet, which can be placed conforming to the waist shape of pet, and can improve the fitting quality.

### [REFERENCE SIGNS LIST]

1, 1X, 1B, 1C, 1D, 1E, 1F:Disposable diaper for pet,
2:Main body portion
2E1: main-body first end
2E2: main-body second end
2E3: main-body third end
2E4: main-body fourth end
2P: inner face
2Q: outer face
10:Topsheet
20:Backsheet
21: back film
22: nonwoven back fabric
30: absorbent core
40,40B,40C:Binding portion
50,50X,50A,50B,50C,50D,50E,50F,50G,50H,50I,50J,50K,50L:Mark portions
53,53X,53A:head-side mark portion
54,54X,54A:crotch-side mark portion
T: thickness direction
W:Waistline direction
Z:Cross direction

## Claims

1. A disposable diaper for pet (1), comprising:
a waistline direction (W) disposed along a waistline direction of a pet;
a cross direction (Z) orthogonal to the waistline direction;
a main body portion (2) having a topsheet, a backsheet, and an absorbent core arranged between the topsheet and the backsheet; and
a binding portion (40) that binds opposing parts of the main body portion when wrapped around a waist of the pet in a wearing state,
the main body portion including:
a main-body first end (2E1) which forms one end in the waistline direction;
a main-body second end (2E2) which forms the other end in the waistline direction,
and arranged an outside of the disposable diaper in the wearing state;
a main-body third end (2E3) which forms one end in the cross direction, and arranged on a head-side of the pet in the wearing state;
a main-body fourth end (2E4) which forms the other end in the cross direction, and
arranged on a crotch-side of the pet in the wearing state; and
a mark portion (50) being designed visible from a side of the backsheet of the disposable diaper for pet, and to indicate a position of the main-body second end in the wearing state,
the mark portion including:
a head-side mark portion (53) residing on a side of the main-body third end of a center in the cross direction of the main body portion; and
a crotch-side mark portion (54) residing on a side of the main-body fourth end of the center in the cross direction of the main body, **characterized in that**
the head-side mark portion is positioned inside or outside, in the waistline direction, of the crotch-side mark portion.

2. The disposable diaper for pet according to claim 1, wherein the binding portion is arranged on a face of a backsheet side of the main-body first end, and bound to a face of a topsheet side of the main-body second end in the wearing state, and at least either the head-side mark portion or crotch-side mark portion is arranged in a region adjacent to the binding portion.

3. The disposable diaper for pet according to any one of claims 1 to 2, wherein the binding portion is arranged on a face of a backsheet side of the main-body first end, and bound to a side of a topsheet side of the main-body second end in the wearing state, and
at least either the head-side mark portion or crotch-side mark portion is arranged in a region overlapping the binding portion.

4. The disposable diaper for pet according to any one of claims 1 to 3, wherein at least either the head-side mark portion or the crotch-side mark portion indicates a position of an outer edge in the waistline direction of the main-body second end in the wearing state.

5. The disposable diaper for pet according to any one of claims 1 to 4, wherein
the binding portion is arranged on the main-body first end, and
the mark portion is formed of the binding portion.

6. The disposable diaper for pet according to claim 5, wherein
the binding portion has a head-side binding portion (40C1) that composes the head-side mark portion, and a crotch-side binding portion (40C2) that composes the crotch-side mark portion, and
the head-side binding portion and the crotch-side binding portion are arranged in the waistline direction with a space in between.

7. The disposable diaper for pet according to any one of claims 1 to 5, wherein the head-side mark portion and the crotch-side mark portion are connected and extend in the cross direction, obliquely to the cross direction.

8. The disposable diaper for pet according to any one of claims 1 to 4 and 7, wherein the mark portion is formed of an outer edge on a side of the main-body first end of the absorbent core.

9. A method for manufacturing a disposable diaper for pet (1), the disposable diaper for pet comprising the features of claim 1:
the method comprising:
a sheet feeding step of feeding a continuous sheet having sheet components arrayed, the sheet components composing any one of the topsheet, the backsheet and
a core wrap;
a marking step of giving the mark portion on the continuous sheet; and
a core laminating step of laminating the absorbent core on the continuous sheet having the mark portion,
the core laminating step being designed to detect a position of the mark portion on the continuous sheet, and to adjust a position of lamination of the absorbent core referring to the position of the mark portion.

## Patentansprüche

1. Wegwerfwindel für Haustiere (1), umfassend:
eine Taillenrichtung (W), die entlang einer Taillenrichtung eines Haustiers angeordnet ist;
eine Querrichtung (Z), die zu der Taillenrichtung orthogonal steht;
einen Hauptkörperabschnitt (2), der ein Deckvlies, ein Rückseitenvlies und einen absorbierenden Kern, der zwischen dem Deckvlies und dem Rückseitenvlies angeordnet ist, aufweist; und
einen Verbindungsabschnitt (40), der gegenüberliegende Teile des Hauptkörperabschnitts verbindet, wenn er in einem Tragezustand um eine Taille des Haustiers gewickelt ist,
wobei der Hauptkörperabschnitt umfasst:
ein erstes Hauptkörperende (2E1), das ein Ende in der Taillenrichtung bildet;
ein zweites Hauptkörperende (2E2), welches das andere Ende in der Taillenrichtung bildet und im Tragezustand an einer Außenseite der Wegwerfwindel angeordnet ist;
ein drittes Hauptkörperende (2E3), das ein Ende in der Querrichtung bildet und im Tragezustand auf einer Kopfseite des Haustiers angeordnet ist;
ein viertes Hauptkörperende (2E4), welches das andere Ende in der Querrichtung bildet und im Tragezustand auf einer Schrittseite des Haustiers angeordnet ist; und
einen Markierungsabschnitt (50), der dazu ausgelegt ist, von einer Seite des Rückseitenvlieses der Wegwerfwindel für Haustiere sichtbar zu sein und eine Position des zweiten Hauptkörperendes im Tragezustand anzugeben,
wobei der Markierungsabschnitt umfasst:
einen kopfseitigen Markierungsabschnitt (53), der auf einer Seite des dritten Hauptkörperendes einer Mitte in der Querrichtung des Hauptkörperabschnitts liegt; und
einen schrittseitigen Markierungsabschnitt (54), der auf einer Seite des vierten Hauptkörperendes der Mitte in der Querrichtung des Hauptkörpers liegt,
**dadurch gekennzeichnet, dass** der kopfseitige Markierungsabschnitt in der Taillenrichtung innerhalb oder außerhalb des schrittseitigen Markierungsabschnitts positioniert ist.

2. Wegwerfwindel für Haustiere nach Anspruch 1, wobei der Verbindungsabschnitt auf einer Fläche einer Rückseitenvliesseite des ersten Hauptkörperendes angeordnet ist und im Tragezustand mit einer Fläche einer Deckvliesseite des zweiten Hauptkörperendes verbunden ist, und
mindestens entweder der kopfseitige Markierungsabschnitt oder der schrittseitige Markierungsabschnitt in einem Bereich neben dem Verbindungsabschnitt angeordnet ist.

3. Wegwerfwindel für Haustiere nach einem der Ansprüche 1 bis 2, wobei der Verbindungsabschnitt auf einer Fläche einer Rückseitenvliesseite des ersten Hauptkörperendes angeordnet ist und im Tragezustand mit einer Seite einer Deckvliesseite des zweiten Hauptkörperendes verbunden ist, und
mindestens entweder der kopfseitige Markierungsabschnitt oder der schrittseitige Markierungsabschnitt in einem Bereich angeordnet ist, der sich mit dem Verbindungsabschnitt überlappt.

4. Wegwerfwindel für Haustiere nach einem der Ansprüche 1 bis 3, wobei mindestens entweder der kopfseitige Markierungsabschnitt oder der schrittseitige Markierungsabschnitt im Tragezustand eine Position eines äußeren Randes in der Taillenrichtung des zweiten Hauptkörperendes angibt.

5. Wegwerfwindel für Haustiere nach einem der Ansprüche 1 bis 4, wobei der Verbindungsabschnitt an dem ersten Hauptkörperende angeordnet ist, und der Markierungsabschnitt aus dem Verbindungsabschnitt besteht.

6. Wegwerfwindel für Haustiere nach Anspruch 5, wobei
der Verbindungsabschnitt einen kopfseitigen Verbindungsabschnitt (40C1), der den kopfseitigen Markierungsabschnitt bildet, und einen schrittseitigen Verbindungsabschnitt (40C2), der den schrittseitigen Markierungsabschnitt bildet, aufweist, und
der kopfseitige Verbindungsabschnitt und der schrittseitige Verbindungsabschnitt in der Taillenrichtung mit einem Zwischenraum dazwischen angeordnet sind.

7. Wegwerfwindel für Haustiere nach einem der Ansprüche 1 bis 5, wobei der kopfseitige Markierungsabschnitt und der schrittseitige Markierungsabschnitt verbunden sind und sich in der Querrichtung schräg zur Querrichtung erstrecken.

8. Wegwerfwindel für Haustiere nach einem der Ansprüche 1 bis 4 und 7, wobei der Markierungsabschnitt aus einem äußeren Rand auf einer Seite des ersten Hauptkörperendes des absorbierenden Kerns besteht.

9. Verfahren zum Herstellen einer Wegwerfwindel für Haustiere (1), wobei die Wegwerfwindel für Haustiere die Merkmale von Anspruch 1 umfasst:
wobei das Verfahren umfasst:
einen Vlieszuführungsschritt, der darin besteht, ein durchgehendes Vlies zuzuführen, auf dem Vlieskomponenten eingerichtet sind, wobei die Vlieskomponenten eines von dem Deckvlies, dem Rückseitenvlies und einem Wickelkern bilden;
einen Markierungsschritt, der darin besteht, den Markierungsabschnitt auf dem durchgehenden Vlies anzubringen; und
einen Kernlaminierungsschritt, der darin besteht, den absorbierenden Kern auf dem durchgehenden Vlies mit dem Markierungsabschnitt zu laminieren,
wobei der Kernlaminierungsschritt dazu ausgelegt ist, eine Position des Markierungsabschnitts auf dem durchgehenden Vlies zu erfassen, und eine Position der Laminierung des absorbierenden Kerns mit Bezug auf die Position des Markierungsabschnitts anzupassen.

## Revendications

1. Couche jetable pour animal de compagnie (1), comprenant :
une direction de la taille (W) disposée le long d'une direction de la taille d'un animal de compagnie ;
une direction transversale (Z) orthogonale à la direction de la taille ;
une partie de corps principal (2) ayant une feuille supérieure, une feuille inférieure et
une âme absorbante disposée entre la feuille supérieure et la feuille inférieure ; et
une partie de liaison (40) qui lie des parties opposées de la partie de corps principal lorsqu'elle est enroulée autour d'une taille de l'animal de compagnie dans un état de port,
la partie de corps principal comportant :
une première extrémité de corps principal (2E1) qui forme une extrémité dans la direction de la taille ;
une deuxième extrémité de corps principal (2E2) qui forme l'autre extrémité dans la direction de la taille, et agencée à l'extérieur de la couche jetable dans l'état de port ;
une troisième extrémité de corps principal (2E3) qui forme une extrémité dans la direction transversale, et agencée sur un côté tête de l'animal de compagnie dans l'état de port ;
une quatrième extrémité de corps principal (2E4) qui forme l'autre extrémité dans la direction transversale, et agencée sur un côté entrejambe de l'animal de compagnie dans l'état de port ; et
une partie repère (50) étant conçue visible depuis un côté de la feuille inférieure de la couche jetable pour animal de compagnie, et pour indiquer une position de la deuxième extrémité de corps principal dans l'état de port,
la partie repère comportant :
une partie repère côté tête (53) résidant sur un côté de la troisième extrémité de corps principal d'un centre dans la direction transversale de la partie de corps principal ; et une partie repère côté entrejambe (54) résidant sur un côté de la quatrième extrémité de corps principal du centre dans la direction transversale du corps principal,
**caractérisée en ce que**
la partie repère côté tête est positionnée à l'intérieur ou à l'extérieur, dans la direction de la taille, de la partie repère côté entrejambe.

2. Couche jetable pour animal de compagnie selon la revendication 1, dans laquelle la partie de liaison est agencée sur une face d'un côté feuille inférieure de la première extrémité de corps principal, et liée à une face d'un côté feuille supérieure de la deuxième extrémité de corps principal dans l'état de port, et
au moins soit la partie repère côté tête soit la partie repère côté entrejambe est agencée dans une région adjacente à la partie de liaison.

3. Couche jetable pour animal de compagnie selon l'une quelconque des revendications 1 et 2, dans laquelle la partie de liaison est agencée sur une face d'un côté feuille inférieure de la première extrémité de corps principal, et liée à un côté d'un côté feuille supérieure de la deuxième extrémité de corps principal dans l'état de port, et
au moins soit la partie repère côté tête soit la partie repère côté entrejambe est agencée dans une région chevauchant la partie de liaison.

4. Couche jetable pour animal de compagnie selon l'une quelconque des revendications 1 à 3, dans laquelle au moins soit la partie repère côté tête soit la partie repère côté entrejambe indique une position d'un bord extérieur dans la direction de la taille de la deuxième extrémité de corps principal dans l'état de port.

5. Couche jetable pour animal de compagnie selon l'une quelconque des revendications 1 à 4, dans laquelle la partie de liaison est agencée sur la première extrémité de corps principal, et
la partie repère est formée de la partie de liaison.

6. Couche jetable pour animal de compagnie selon la revendication 5, dans laquelle la partie de liaison a une partie de liaison côté tête (40C1) qui compose la partie repère côté tête, et une partie de liaison côté entrejambe (40C2) qui compose la partie repère côté entrejambe, et
la partie de liaison côté tête et la partie de liaison côté entrejambe sont agencées dans la direction de la taille avec un espace entre elles.

7. Couche jetable pour animal de compagnie selon l'une quelconque des revendications 1 à 5, dans laquelle la partie repère côté tête et la partie repère côté entrejambe sont reliées et s'étendent dans la direction transversale, obliquement par rapport à la direction transversale.

8. Couche jetable pour animal de compagnie selon l'une quelconque des revendications 1 à 4 et 7, dans laquelle la partie repère est formée d'un bord extérieur sur un côté de la première extrémité de corps principal de l'âme absorbante.

9. Procédé de fabrication d'une couche jetable pour animal de compagnie (1), la couche jetable pour animal de compagnie comprenant les caractéristiques selon la revendication 1 :
le procédé comprenant :
une étape d'alimentation en feuilles consistant à alimenter une feuille continue ayant des composants de feuille disposés en réseau, les composants de feuille composant l'une quelconque de la feuille supérieure, de la feuille inférieure et d'une enveloppe centrale ;
une étape de repérage consistant à donner la partie repère sur la feuille continue ; et une étape de stratification d'âme consistant à stratifier l'âme absorbante sur la feuille continue ayant la partie repère,
l'étape de stratification d'âme étant conçue pour détecter une position de la partie repère sur la feuille continue, et pour ajuster une position de stratification de l'âme absorbante en se référant à la position de la partie repère.
